# EUROPEAN PATENT APPLICATION

(11) **EP 2 835 123 A1**
(43) Date of publication of application: **11.02.2015**
(21) Application number: 13179752.4
(22) Date of filing: 08.08.2013
(51) Int. Cl.: A61F 13/49, A61F 13/494

(54) **Cuff connector material**

(71) Applicant: The Procter & Gamble Company, Cincinnati, OH 45202 (US)
(72) Inventor: Martynus, Cornelia Beate, 61130 Nidderau-Ostheim (DE); Borbach, Dr., Markus, 60487 Frankfurt (DE)
(74) Representative: Borbach, Markus

(57) **Abstract**

The invention relates to an infant or adult diaper, having a first and second elastic cuff (30a, 30b) being positioned on either longitudinal side of the topsheet (22) and opposing each other, the longitudinally extending elastic cuffs (30a, 30b) each having a proximal edge (32) being attached to the diaper and each having a distal edge (34), at least the crotch region (B') of which is not attached to the diaper (10) and forms a free flap (38a, 38b); wherein the diaper (10) comprises a cuff connector (70) being attached to the first longitudinally extending elastic cuff (30a) and to the second longitudinally extending elastic cuff (30b), the cuff connector (70) extending in a generally transverse direction and being able to extert a force between the longitudinally extending elastic cuffs (30a, 30b) wherein the cuff connector (70) comprises a cuff connector material and the cuff connector material:
- has a transverse strain of about 0.8 at a first cycle extension force of about 1.0 N or less, as measured according to the in-use two-cycle hysteresis test; and
- has about 30% set or less after having been extended to a transverse strain of about 0.8, as measured according to the in-use two-cycle hysteresis test; and
- is elastically extensible in the transverse direction to a strain of at least about 0.8, as measured according to the in-process two-cycle hysteresis test at 22°C

## Description

### FIELD OF THE INVENTION

This invention is directed to diapers, including infant diapers, training pants, and adult incontinence articles, and the like having an improved fit and leakage protection. In one aspect, the improved fit and leakage protection is achieved by a connector connecting transversely opposite cuffs.

### BACKGROUND OF THE INVENTION

Several diaper types have been proposed with components to improve fit and / or reduce leakage of feces and urine from the diaper, to reduce soiling of the genitals or other skin by the feces, or to reduce mixing of urine and feces, to further reduce the risk of irritation of the skin. For example, diapers with a topsheet with an opening, providing a passageway to a void space for collected feces and urine have been proposed; also proposed are diapers with two openings to receive the urine and feces in separate areas; also proposed are diapers with a transversely positioned three-dimensional resilient barrier wall or partition placed in the centre of the diaper, to receive feces and urine respectively on either side of said wall or partition, and to avoid migration of the feces to the front of the article.

For example, EP 1 219 274 B1 (Tabata et al.) discloses a disposable absorbent article with at least one standing cuff. Such a standing cuff requires certain stretching members and therefore appear complex and costly in production.

EP 1 232 736 B1 (Toyoshima et al.) discloses an absorbent article with a pair of three-dimensional guards and a plurality of associated elastic members. Also there three-dimensinal guards appear complex and costly in production. Further, they might comprise the wearing comfort of the absorbent article in some way and might make it more difficult for a caretaker to properly place the article on a wearer.

The inventors found however that, conventional cuffs and in particular relatively high conventional cuffs can be used in combination with certain cuff connections. When used with such connectors even high cuffs provide a reliable leakage protection and are high wearing comfort.

### SUMMARY OF THE INVENTION

The invention relates to an infant or adult diaper. More particularly, the inventin relates to an infant or adult diaper (10) having a longitudinal centerline (Y) and a lateral centerline (X) perpendicular to the longitudinal centerline (Y), wherein the diaper (10) comprises a topsheet (22) and a backsheet (24) and positioned therein between an absorbent core (20), and a first longitudinally extending elastic cuff (30a) and second longitudinally extending elastic cuff (30b), the first and the second elastic cuff (30a, 30b) being positioned on either longitudinal side of the topsheet (22) and opposing each other, and wherein the diaper (10) has a crotch region (B), positioned in between a front region (A) and a back region (C) and the first and the second longitudinally extending elastic cuff (30a, 30b) each have a crotch region (B'), positioned in between a front region (A') and a back region (C'), these regions of the longitudinally extending elastic cuffs (30a, 30b) being generally positioned in or adjacent to the respective regions of the diaper (10), and the first longitudinally extending elastic cuff (30a) and the second longitudinally extending elastic cuff (30b) each have a proximal edge (32), at which the respective longitudinally extending elastic cuff is attached to the diaper and the the first longitudinally extending elastic cuff (30a) and the second longitudinally extending elastic cuff (30b) each have a distal edge (34), at least the crotch region (B') of which is not attached to the diaper (10) and forms a free flap (38a, 38b) and wherein the diaper (10) comprises a cuff connector (70) being attached to the first longitudinally extending elastic cuff (30a) and the second longitudinally extending elastic cuff (30b), extending in a generally transverse direction and being able to extert a force between the first longitudinally extending elastic cuff (30a) and the second longitudinally extending elastic cuff (30b), wherein the cuff connector (10) comprises a cuff connector material and the cuff connector material:
- has a transverse strain of about 0.8 at a first cycle extension force of about 1.0N or less, as measured according to the in-use two-cycle hysteresis test; and
- has about 30% set or less after having been extended to a transverse strain of about 0.8, as measured according to the in-use two-cycle hysteresis test; and
- is elastically extensible in the transverse direction to a strain of at least about 0.8, as measured according to the in-process two-cycle hysteresis test at 22°C

### BRIEF DESCRIPTION OF THE DRAWINGS

Figure 1 shows a top view of a diaper in flat state suitable for being used with features of the present invention, but not embodying the present invention.
Figure 2 shows a cross-sectional view of the diaper of Figure 1 taken at the section line 2-2.
Figure 3 shows a top view of a diaper of the present invention in flat state.
Figure 4 shows a cross-sectional view of the diaper of Figure 3 taken at the section line 4-4.
Figure 5 shows a perspective view of the diaper of Figures 3 and 4.

### DETAILED DESCRIPTION OF THE INVENTION

"Diaper" is used herein, to refer to absorbent articles to be placed about the wearer's lower torso, and include infant (baby and toddler) diapers and adult diapers, and it includes so-called diapers with fasteners, to be fastened around the lower torso, and pant-type diapers.

"Front region" (A) and 'back region' (C) are used herein to refer to the two regions, which are in use, respectively, the closest to the front of the wearer and the back of the wearer, each spanning the transverse dimension of the diaper or diaper portion or element thereof, e.g. elastic cuff or topsheet, and each region having a longitudinal dimension that is (exactly or about or around) one fourth of the longitudinal dimension of the diaper or diaper portion or element thereof, e.g. elastic cuff or topsheet.

"Crotch region" (B) is used herein to refer to the region positioned between the front region and the back region, having also a longitudinal dimension that is (exactly or about or around) one half of the longitudinal dimension of the diaper, diaper portion or element thereof, e.g. elastic cuff or topsheet.

"Longitudinal" is used herein to refer to the direction which is running substantially parallel or exactly parallel to the longitudinal centerline (Y) of the diaper or diaper portion or topsheet, which may be the machine direction (MD) of the process.

"Lateral" or "transverse" is used herein to refer to the direction which is substantially perpendicular or exactly perpendicular to the longitudinal centerline (Y) of the diaper or diaper portion or topsheet, which may be the cross-machine direction of the process (CD).

"Z-direction" is used herein to refer to the direction perpendicular to the longitudinal direction and perpendicular to the transverse or lateral direction.

"Longitudinal dimension", "transverse dimension" or "Z-dimension" is used herein to refer to the dimension of the diaper, diaper portion or element thereof, e.g. elastic cuff or topsheet, which is measured respectively in the longitudinal direction, transverse direction or Z-direction of the diaper or diaper portion or element thereof.

"Substantially perpendicular" or "substantially parallel" is used herein to refer to directions within 30° or 20° or 10° or 5° from the exact perpendicular or parallel direction, unless stated or specified otherwise.

As used herein, "along" means 'at least partially substantially parallel to and adjacent to'.

"Relaxed" or "relaxed state" or "contracted" or "contracted state" is used herein to refer to the state of the diaper or diaper portion wherein no forces are applied to respectively the diaper or diaper portion.

"Flat state" is used herein to refer to the state of the diaper when the diaper is laid out flat onto an even horizontal surface.

"Distal edge" is used herein to refer to the longitudinally extending edge of an elastic cuff which in use is closer to the wearer's body than the other longitudinally extending edge of the same elastic cuff.

A "nonwoven web" as used herein means a manufactured sheet, web or batt of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yam). Nonwoven webs can be formed by many processes such as meltblowing, spunbonding, solvent spinning, electrospinning, carding and airlaying. The basis weight of nonwoven webs is usually expressed in grams per square meter (g/m2 or gsm).

The invention will be further explained with reference to the figures. Additionally, the invention will be explained with regard to certain of its features. The invention relates to a diaper comprising *inter alia* a topsheet, a backsheet, longitudinally extending elastic cuffs, referred to also as barrier leg cuffs, elastic waist features and a cuff connector. These elements will now first be described in some further detail.

### Cuff connector

The cuff connector may have a transverse dimension in relaxed state of from 3.0 cm, or from 4.0 cm, or from 5.0 cm, to for example 15.0 cm, or to 10.0 cm, or to 8.0 cm or to 7.0 cm, or to 6.0 cm or to 5.5 cm.

The cuff connector may have an average longitudinal dimension in relaxed state of at least 2.0 mm, or at least 4.0 mm, or at least 5.0 mm to less than 25.0 mm, 20.0 mm, 15.0 mm, 12.0 mm or less than 10.0 mm.

If the cuff connector is provided in the form of the strip, the above longitudinal and transverse dimensions are those of that strip.

It is useful to position the cuff connector in the crotch area of the diaper, for example in the center thereof and a bit closer to the front region of the diaper. The distance from the front edge of the diaper to the edge of the cuff connector facing the front edge of the diaper may therefore be chosen to be 25% to 50% or 30 % to 45% of the to total length of the diaper taken from the front edge to the rear edge of the diaper.

The cuff connector is elastically extensible in at least the transverse direction, i.e. is able to extend upon application of a force, and to contract to about its original width upon release of said force.

The cuff connector may also be elastically extensible in the longitudinal direction.

The cuff connector may be made of an elasticated sheet material, rendering the cuff connector elastically extensible in the transverse direction at least.

"Elasticated" when used herein for the cuff connector means that it is made of an inelastic sheet material that has been provided only in a specific area or in specific areas, i.e. the elasticated area(s), with an elastic material, e.g. one or more elastic bands or strands.

One or more portions of the cuff connector may be elasticated by application of one or more elastic band(s) or strand(s) extending substantially in transverse direction to a sheet material that may be inelastic e.g. an inelastic nonwoven sheet.

The nonwoven sheet material may be made of polyolefins known in the art, such as polyethylene and/or polypropylene, made into fibers, including bicomponent fibers that are then made into a nonwoven sheet. The nonwoven sheet material may be a necked nonwoven. The nonwoven sheet material may be a meltblown nonwoven or spunbond nonwoven or carded nonwoven; it may be a laminate thereof; for example it may be a laminate of spunbond or carded layer or layers and meltblown nonwoven layer(s).

The cuff connector material may be a barrier material; it may be hydrophobic; e.g. it may be hydrophilic and made hydrophobic with a hydrophobic surface coating, such as known in the art, for example a wax or a hydrophobic surface coating comprising one or more silicone polymers or fluorinated polymers. The cuff connector may also be treated with a lotion.

### Cuff connector material

The cuff connector comprises a cuff connector material. The cuff connector may be essentially or fully provided by the material. The material can be a non-woven or a laminate, as mentioned above. Cuff connector materials fulfilling certain criteria, as expressed below, have been found to work best. Certain strain measurements are therefore useful for identifying a beneficial material.

The cuff connector material has a transverse strain of 0.8 (extension to at least 1.8 times its width) at a first cycle extension force of 1.0 N or less, as measured according to the in-use two-cycle hysteresis test as described herein below. The cuff connector material may have a transverse strain of 1.2 (extension to at least 2.2 times its width) at a first cycle extension force of 1.5 N or less, or at a first cycle extension force of 1.0N or less, as measured according to the in-use two-cycle hysteresis test. The cuff connector material may have a transverse strain of 0.8 or 1.2 at a first cycle extension force of 0.8 N or less, or at a first cycle extension force of 0.6 N or less, but typically at a first cycle extension force of at least 0.2 N, or at least 0.3 N, as measured according to the in-use two-cycle hysteresis test. Hence, the cuff connector material is such that when the wearer spreads the legs, the elastic cuffs 30a, 30b can expand and move outwards without being too restricted by the cuff connector material's forces. This ensures the elastic cuffs 30a, 30b can stay in place and reduces the risk of feces leakage over the elastic cuffs 30a, 30b.

The cuff connector material has 30% set or less after having been extended to a transverse strain of 0.8 , as measured according to the in-use two-cycle hysteresis test. The cuff connector material may have 30% set or less after having been extended to a transverse strain of 1.2, as measured according to the in-use two-cycle hysteresis test. The cuff connector material may have 20% set or less after having been extended to a transverse strain of 0.8 or 1.2, as measured according to the in-use two-cycle hysteresis test. It may have 10% set or less after having been extended to a transverse strain of 0.8 or 1.2, as measured according to the in-use two-cycle hysteresis test. Hence, the transverse cuff connector material is such that in use, when the wearer moves the legs together, the transverse cuff connector material can contract to a very small dimension very quickly, thus avoiding slack in the cuff connector material, and hence reducing the risk of feces leakage over the cuff connector material. Therefore, the cuff connector material remains in contact with the skin of the wearer during use, so both when the wearer moves and spreads the legs and when the wearer rests and closes the legs.

The cuff connector material is elastically extensible in the transverse direction to a strain of at least 0.8, as measured according to the in-process two-cycle hysteresis test. The cuff connector material or cuff connector material 15 may be elastically extensible in the transverse direction to a strain of at least 1.2 or at least 1.5 or at least 2.0 or at least 2.5 or at least 3.0 or at least 3.5, as measured according to the in-process two-cycle hysteresis test. This can be done with any force suitable for processing elastic materials, typically a force of 20 N or less, or typically a force of 10N or less. Therefore, the cuff connector material is such that it can be processed in a process that requires high extension, and that it contracts about immediately to about its original width, to allow high speed of processing.

It should be understood that the cuff connector material is considered as being elastically extensible in the transverse direction to a certain strain value if after having been extended to said strain value according to the in-process two-cycle hysteresis test, the percent set measured according to the in-process two-cycle hysteresis test is of 30% set or less or 20% or less or 10% or less. Having such a low set value ensures that the cuff connector material is not damaged during processing of the diaper 1.

### TEST METHODS

### Two Cycle Hysteresis Tests (In-Use and In-Process):

### Equipment and preparation of samples:

Suitable instruments for this test include tensile testers commercially available from MTS Systems Corp., Eden Prairie, Minn. (e.g. Alliance RT/1 or Sintech 1/S) or from Instron Engineering Corp., Canton, Mass. or from Zwick/Roell, Kennesaw, Georgia/Ulm, Germany.

The tester is equipped with a grip face set in the upper and lower grips, where each set has one rubber-coated face (80 A shore hardness) and one contact line face (metal), with a screw mechanism that engages the contact line face into the rubber-coated face to prevent slippage. The length of the upper and lower grips is at least as long as the clamped length of the specimen to be tested, the grip length being the dimension of the grip parallel to the length of the specimen. A load cell is used so that the maximum load measured is within 10 - 90% of the maximum capacity of the load cell. The instrument is calibrated according to the manufacturer's specification.

Before obtaining the specimen from the diaper, the diaper is kept in a relaxed state at ambient temperature (22°C +/- 5°C) for at least 24 hours.

The cuff connector material is cut to obtain a rectangular specimen having a transverse dimension (width) of 20 mm centered on the longitudinal centerline Y of the diaper and a longitudinal dimension (length) of 30 mm, both transverse and longitudinal dimensions being measured in relaxed, contracted state of the specimen when the specimen is laid out flat onto an even horizontal surface. The transverse dimension and longitudinal dimension of the specimen are measured parallel to respectively the transverse dimension and longitudinal dimension of the cuff connector material.

In-Use Two-Cycle Hysteresis Test (measurement at 34° C): The In-Use two-cycle hysteresis test is performed as follows:
1. Slack Adjustment: Move the crosshead at a speed of 13 mm/minute until the slack adjustment preload force of 0.02 N is achieved. The distance between the lines of contact of the grips at the slack adjustment preload force of 0.02N is the adjusted gauge width W_{GAdj} which is equal to the initial sample width W₀ (engineering strain = 0%).
2. First Cycle:
   The specimen is extended to the tested engineering strain at a crosshead speed of 100 mm/min, and held at this engineering strain for 60 seconds. The crosshead is then returned to the adjusted gauge width W_{GAdj} at a crosshead speed of 100 mm/min, and held at this adjusted gauge width W_{GAdj} for 60 seconds.
3. Second Cycle:
   The specimen is extended to the same engineering strain as the engineering strain in the first cycle, at a crosshead speed of 100 mm/min, and held at this engineering strain for 60 seconds. The crosshead is then returned to the adjusted gauge width W_{GAdj} at a crosshead speed of 100 mm/min, and held at this adjusted gauge width W_{GAdj} for 60 seconds.

The force, time and engineering strain data are acquired for all segments of the in-use two cycle hysteresis test at a frequency of 50 Hz. The percent set (% Set) is defined as the engineering strain, in percent, where the 2^{nd} cycle extension force of 0.069 N (7 gram-force) is measured.

A minimum of 5 specimens are measured, to determine the average test values, average data being provided by the tensile tester test software (e.g. Alliance RT1 MTS Instrument is TestWorks4 version 4.08B)

The method report specifies the tested engineering strain, the specimen dimensions and the clamped length used for the measurements.

The properties of the cuff connector material reported for the method are the 1^{st} cycle extension force at the tested engineering strain and the % set.

### In-Process Two-Cycle Hysteresis Test (measurement at 22°C):

The In-Process two-cycle hysteresis test is performed as follows:
1. Slack Adjustment: Same as step 1 above for the In-Use Two-Cycle Hysteresis Test.
2. First Cycle:
   The specimen is extended to the tested engineering strain at a crosshead speed of 800 mm/min. There is no hold at this engineering strain. The crosshead is then returned to the adjusted gauge width W_{GAdj} at a crosshead speed of 800 mm/min, and held at this engineering strain for 60 seconds.
3. Second Cycle:
   The specimen is extended to the same engineering strain as the engineering strain in the first cycle, at a crosshead speed of 800 mm/min. There is no hold at this engineering strain. The crosshead is then returned to the adjusted gauge width W_{GAdj} at a crosshead speed of 800 mm/min and held at this engineering strain for 60 seconds.

Each specimen of cuff connector material of the diaper is inspected for damage after the In-Process two-cycle hysteresis test. Cuff connector material sheet material of the invention must have no damage after the test. The damage is defined via the percent set. The percent set (% Set) is defined as the engineering strain, in percent, where the 2^{nd} cycle extension force of 0.069 N (7 gram-force) is measured. If the percent set is lower than 30%, the specimen is considered as not being damaged and is therefore considered as being elastically extensible to tested engineering strain.

A minimum of 5 specimens are measured, to determine the average test values, average data being provided by the tensile tester test software (e.g. Alliance RT1 MTS Instrument is TestWorks4 version 4.08B)

The method report specifies the used engineering strain, the specimen dimensions and the clamped length used for the measurements.

The property of the cuff connector material sheet reported for the method is the % set.

### Attachment of the cuff connenctor sheet to the elastic cuffs

Parts of the cuff connector are attached to the elastic cuffs in the front, crotch region or rear region of the longitudinally extending elastic cuffs. A first cuff attachment area and a second cuff attachment area are formed by attaching respectively a first or second end portion of the cuff connector to an area of the longitudinally extending elastic cuffs.

The attachment of the cuff connenctor to the elastic cuffs can be done by adhesive bonding, pressure bonding, ultrasonic bonding or any other attachment mean known in the art. A cuff attachment area may comprise one or more individual bond(s). Such individual bond(s) may be arranged according to a certain bonding pattern.

It should be understood that if an attachment area comprises more than one individual bond, these are herein considered as a single unitary cuff attachment area.

The bonding pattern density of the first and/or second cuff attachment area(s) may be more than 20%, or more than 30%, or more than 40%, or more than 60%, up to 100%.

### Barrier leg cuffs

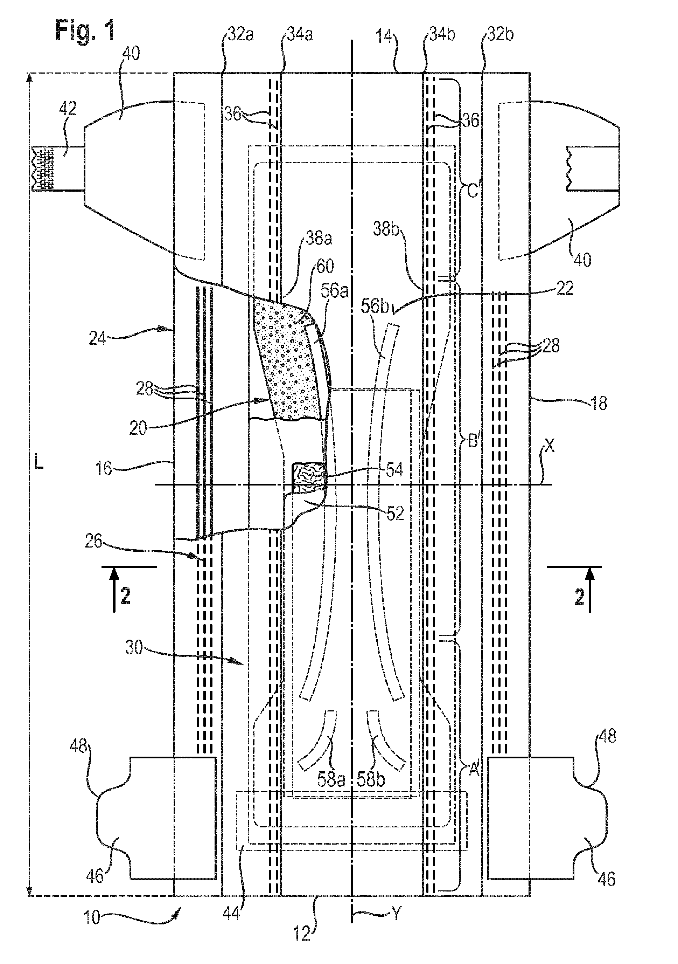

The absorbent article comprises a pair of barrier leg cuffs, also referred to as longitudinally extending elastic cuffs. The barrier leg cuffs can be formed from a piece of material, typically a nonwoven, which is partially bonded to the rest of the article so that a portion of the material, the barrier leg cuffs, can be partially raised away and stand up from the plane defined by the topsheet when the article is pulled flat as shown e.g. in Fig. 1. The barrier leg cuffs can provide improved containment of liquids and other body exudates approximately at the junction of the torso and legs of the wearer. The barrier leg cuffs extend at least partially between the front edge and the back edge of the diaper on opposite sides of the longitudinal axis. The barrier leg cuffs are delimited by a proximal edge joined to the rest of the article, typically the topsheet and/or the backsheet, and a free distal edge, which is intended to contact and form a seal with the wearer's skin. The barrier leg cuffs are joined at the proximal edge with the chassis of the article by a bond which may be made for example by gluing, fusion bonding or combination of known bonding means. The bond at the proximal edge may be continuous or intermittent. The side of the bond closest to the raised section of the leg cuffs delimits the proximal edge of the standing up section of the leg cuffs.

The barrier leg cuffs can be integral with the topsheet or the backsheet, or more typically be formed from a separate material joined to the rest of the article. Typically the material of the barrier leg cuffs may extend through the whole length of the diapers but is "tack bonded" to the topsheet towards the front edge and back edge of the article so that in these sections the barrier leg cuff material remains flush with the topsheet. Each barrier leg cuff 34 may comprise one, two or more elastic strings 35 close to this free terminal edge 66 to provide a better seal.

Each cuff may have an elastic tension of at least 20 grams (0.2N) and up to 100 grams (1.0N), or up to 50 grams (0.5N), when extended to a 95% or 80% extension strain.

The cuffs or cuff web material may comprise a nonwoven sheet material. The same nonwoven sheet materials as the ones used to make the cuff connector may be used. The nonwoven sheet material may be a nonwoven barrier sheet material that is liquid impermeable, as known in the art, including for example nonwoven laminate(s) with one or more spunbond layers and/or carded layers, and one or more meltblown layers. The fibers used to form the nonwoven sheet material may be selected from polypropylene fibers, polyethylene fibers, bicomponent fibers, nano-fibers and any combinations thereof.

The cuffs may have any suitable dimensions, for example depending on the diaper dimensions. They may extend about the full length of the diaper. They may have a transverse dimension perpendicular to longitudinal centerline Y of the diaper of for example at least 30 mm, or for example at least 40 mm.

In addition to the barrier leg cuffs, the article may comprise gasketing cuffs, which are joined to the chassis of absorbent article, in particular the topsheet and/or the backsheet and are placed transversely outwardly relative to the barrier leg cuffs. The gasketing cuffs can provide a better seal around the thighs of the wearer. Usually each gasketing leg cuff will comprise one or more elastic string or elastic element comprised in the chassis of the diaper for example between the topsheet and backsheet in the area of the leg openings.

US3,860,003 describes a disposable diaper which provides a contractible leg opening having a side flap and one or more elastic members to provide an elasticized leg cuff (a gasketing cuff). US4,808,178 and US4,909,803 issued to Aziz et al. describe disposable diapers having "stand-up" elasticized flaps (barrier leg cuffs) which improve the containment of the leg regions. US4,695,278 and US4,795,454 issued to Lawson and to Dragoo respectively, describe disposable diapers having dual cuffs, including gasketing cuffs and barrier leg cuffs. All or a portion of the barrier leg and/or gasketing cuffs may be treated with a lotion.

### Topsheet

The diaper comprises a topsheet.

It should be understood that the topsheet herein may be an individual topsheet or a topsheet that is part of a topsheet web, whereby such web is then subsequently divided into a multitude of individual topsheets. For the purpose of the invention, when referred to topsheet, this shall include a topsheet web, respectively, unless stated otherwise. The same applies for the backsheet and absorbent core (that may be a backsheet web or absorbent core web), as referred to herein.

The topsheet of the diaper herein may be made of any suitable material known in the art, provided it allows urine to pass. Hereto, it may be made of a urine permeable material, including hydrophilic material, or material treated to be hydrophilic. It may be an apertured topsheet which comprise apertures to allow urine to pass to the absorbent core under the topsheet;

The topsheet may be either hydrophilic or hydrophobic.

Preferred topsheet materials are nonwoven materials, including laminates and/or materials with apertures, such as apertured films, aperture formed films.

The topsheet, or for example only the crotch and/or back region thereof, may comprise a skin care composition, e.g. a lotion, as known in the art.

### Fastening system

The absorbent article may include a fastening system. The fastening system can be used to provide lateral tensions about the circumference of the absorbent article to hold the absorbent article on the wearer as is typical for taped diapers. This fastening system is not necessary for training pant article since the waist region of these articles is already bonded. The fastening system usually comprises a fastener such as tape tabs, hook and loop fastening components, interlocking fasteners such as tabs & slots, buckles, buttons, snaps, and/or hermaphroditic fastening components, although any other known fastening means are generally acceptable. A landing zone is normally provided on the front waist region for the fastener to be releasably attached. Some exemplary surface fastening systems are disclosed in US 3,848,594, US4,662,875, US 4,846,815, US4,894,060, US4,946,527, US5,151,092 and US 5,221,274 issued to Buell. An exemplary interlocking fastening system is disclosed in US6,432,098. The fastening system may also provide a means for holding the article in a disposal configuration as disclosed in US 4,963,140 issued to Robertson et al.

The fastening system may also include primary and secondary fastening systems, as disclosed in US4,699,622 to reduce shifting of overlapped portions or to improve fit as disclosed in US5,242,436, US5,499,978, US5,507,736, and US5,591,152.

As part of the fastening system, the absorbent article may comprise front ears and back ears as is known in the art. The ears can be integral part of the chassis, for example formed from the topsheet and/or backsheet as side panel. Alternatively, they may be separate elements attached by gluing and / or heat embossing or pressure bonding. The back ears are advantageously stretchable to facilitate the attachment of the tabs on the landing zone and maintain the taped diapers in place around the wearer's waist. The back ears may also be elastic or extensible to provide a more comfortable and contouring fit by initially conformably fitting the absorbent article to the wearer and sustaining this fit throughout the time of wear well past when absorbent article has been loaded with exudates since the elasticized ears allow the sides of the absorbent article to expand and contract.

### Elastic waist feature

The absorbent article may also comprise at least one elastic waist feature (not represented) that helps to provide improved fit and containment. The elastic waist feature is generally intended to elastically expand and contract to dynamically fit the wearer's waist. The elastic waist feature preferably extends at least longitudinally outwardly from at least one waist edge of the absorbent core 28 and generally forms at least a portion of the end edge of the absorbent article. Disposable diapers can be constructed so as to have two elastic waist features, one positioned in the front waist region and one positioned in the back waist region. The elastic waist feature may be constructed in a number of different configurations including those described in US4,515,595, US4,710,189, US5,151,092 and US 5,221,274.

If should be noted, that the design (including the dimensions) of the cuffs, also the gasketing cuffs, and of the elastic waist feature and the fastening system has an influence on the overall fit of the diaper when worn and therefore on the position and functioning of the cuff connector. Where elastics are used in the various portions of the diaper, their elastic behavious also has such an influence.

### Relations between the layers

Typically, adjacent layers and components will be joined together using conventional bonding method such as adhesive coating via slot coating or spraying on the whole or part of the surface of the layer, or thermo-bonding, or pressure bonding or combinations thereof. This bonding is not represented in the Figures (except for the bonding between the raised element of the leg cuffs with the topsheet) for clarity and readability but bonding between the layers of the article should be considered to be present unless specifically excluded. Adhesives may be typically used to improve the adhesion of the different layers, for example between the backsheet and the core wrap. The glue may be any standard hotmelt glue as known in the art.

### Method of making the article

The absorbent articles of the invention may be made by any conventional methods known in the art. In particular the articles may be hand-made or industrially produced at high speed

### Backsheet

The backsheet of the diaper may be liquid impervious, as known in the art. The liquid impervious backsheet preferably comprises a thin plastic film such as a thermoplastic film, for example having a thickness of about 0.01 mm to about 0.05 mm. Suitable backsheet materials comprise typically breathable material, which permit vapors to escape from the diaper while still preventing exudates from passing through the backsheet. Suitable backsheet films include those manufactured by Tredegar Industries Inc. of Terre Haute, IN and sold under the trade names X15306, X10962 and X10964.

The backsheet, or any portion thereof, may be elastically extendable in one or more directions. The backsheet may be attached or joined to a topsheet, the absorbent core, or any other element of the diaper by any attachment means known in the art. It may be highly preferred that the longitudinal side edges of the topsheet and backsheet are directly attached to one another.

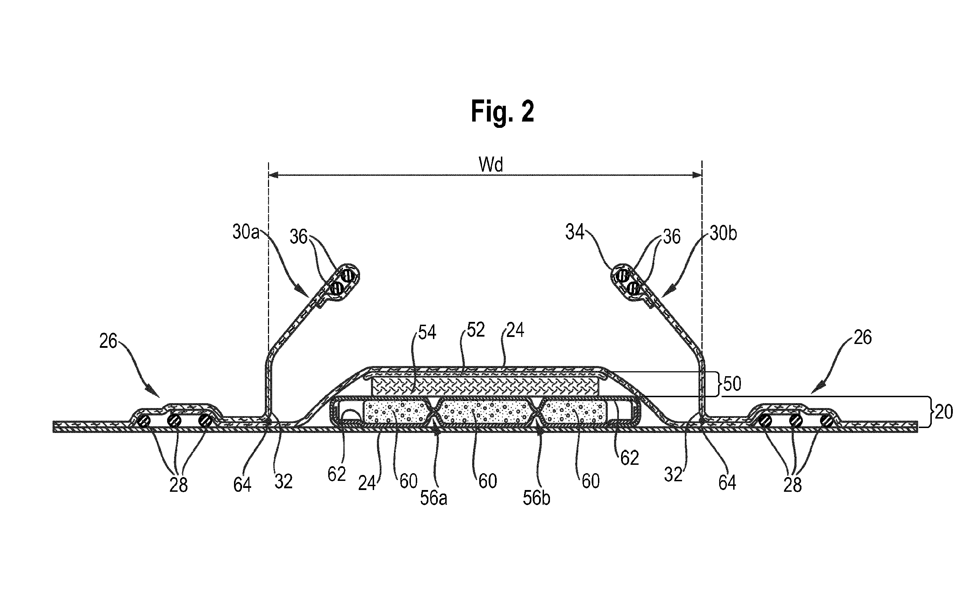

The essential elements of diaper 10, for which the present invention can be useful, are shown in Figures 1 and 2. Notably, the respective diaper does not already include the features of the present invention, however, serves to illustrate the benefits achievable by the present invention, once the invention is used for a diaper such as shown in Figure 1 and Figure 2.

The diaper 10 comprises a front edge 12 and a rear edge 14. The diaper also has a left side edge 16 and a right side edge 18. For the purpose of absorbing liquids, the diaper comprises absorbent core 20. The absorbent core 20 is positioned between a topsheet 22 and a back sheet 24. The topsheet 22 is positioned on the wearer facing side of the diaper 10 and will allow, at least in one portion, the passage of liquids. The backsheet 24, however, is normally liquid proof (but may be vapor permeable).

The diaper 10 comprises gasketing cuffs 26, arranged on the wearer facing side, which is shown in the view of Figure 1. The gasketing cuffs 26 comprise elastics 28. These elastics 28 are normally arranged between topsheet 22 and backsheet 24. Alternatively, the elastics 28 can be provided between an extention sheet of the barrier leg cuffs 30 and the backsheet 24. The extension sheet can be joined to or intregral with the barrier leg cuff(s). The elastics 28 can also be provided in the form of lines of elastic adhesives.

The diaper 10 also comprises barrier leg cuffs 30 (also referred to as "BLCs" or longitudinally extending cuffs). These barrier leg cuffs 30 are readibly visible on the barrier facing side of the diaper, which is shown in the view of Figure 1. The barrier leg cuffs 30 exhibit a proximal edge 32 and a distal edge 34. The proximal edge 32 extends along the longitudinal direction of the diaper. Typically, the barrier leg cuffs 30 are attached to the diaper 10 at least along their proximal edge 32. Normally, at least in the vicinity of front edge 12 and rear edge 14 further attachment areas are present. The barrier leg cuffs 30 also each have a distal edge 34, which also extends in the longitudinal direction.

The barrier leg cuffs 30 are equiped with elastics 36 positioned adjacent to distal edge 34. By means of these elastics 36, the barrier leg cuffs 30 can be brought in good contact with a wearer and will remain in contact with the wearer while the diaper 10 is worn. A portion of the barrier leg cuffs 30 is normaly not attached to other portions of the diaper 10 and therefore forms a free flap 38. Such a free flap 38 is typically provided at least in the crotch portion B' of the barrier leg cuff 30. Typically, two longitudinally extending barrier leg cuffs are provided on either side of a diaper. As shown in Figure 1, these are barrier leg cuffs 30a and 30b. Each cuff has a respective proximal edge 32a, 32b and a respective distal edge 34a, 34b. Further, each cuff is provided with a free flap 38a, 38b. The barrier leg cuffs and in particular their free flaps 38a, 38b are provided in the form of mirror images and are centered about the longitudinal axis Y of the diaper.

The diaper 10 is also equipped with a fastening system. In the back region C of the diaper, the fastening system comprises back ears 40. The back ears 40 are provided with adhesive tapes 42. A landing zone 44 for the adhesive tapes 42 is provided in a region of the backsheet 24. When the diaper 10 is placed around the lower torso of a wearer, the back ears 40 co-operate with front ears 46. The front ears 46 comprise a tab portion 48.

Figure 2 provides a cross section view of the diaper as indicated by line 2-2 in Figure 1. This cross sectional view, provides more details about the structure of the core 20. Underneath topsheet 22, an acquisition distribution system 50 is provided. This system comprises an acquisition layer 52, which is placed adjacent to or in proximity of topsheet 22. Underneath the acquisition layer 52 a distribution layer 54, serving for the distribution of liquid, is arranged. Underneath the acquisition distribution system 50, core 20 is placed. As visible in this view, the core 20 comprises a number of channels 56. Also, several channels of different dimensions can be provided, such as further channel 58 as visible in Figure 1. Between such channels absorbent material 60 is arranged. The absorbent material 60 is covered by core wrap 62.

It is also readibly visible from Figure 2, that dedicated attachment means are provided at the proximal edge 32 of the barrier leg cuff 30. As shown, this attachment means can be provided in the form of a barrier leg cuff bond 64. A respective bond can be provided by a longitudinally extending line of glue.

With regard to the diaper 10 shown in Figure 1 and Figure 2, which represents a modem diaper (not embodying the present invention), it has become apparent, that the distal edges 34a and 34b of the barrier cuffs 30 have a high degree of freedom of movement. The present invention has benefits in limiting this freedom of movement. This can lead to a better and more reproducible fit, especially in the crotch region.

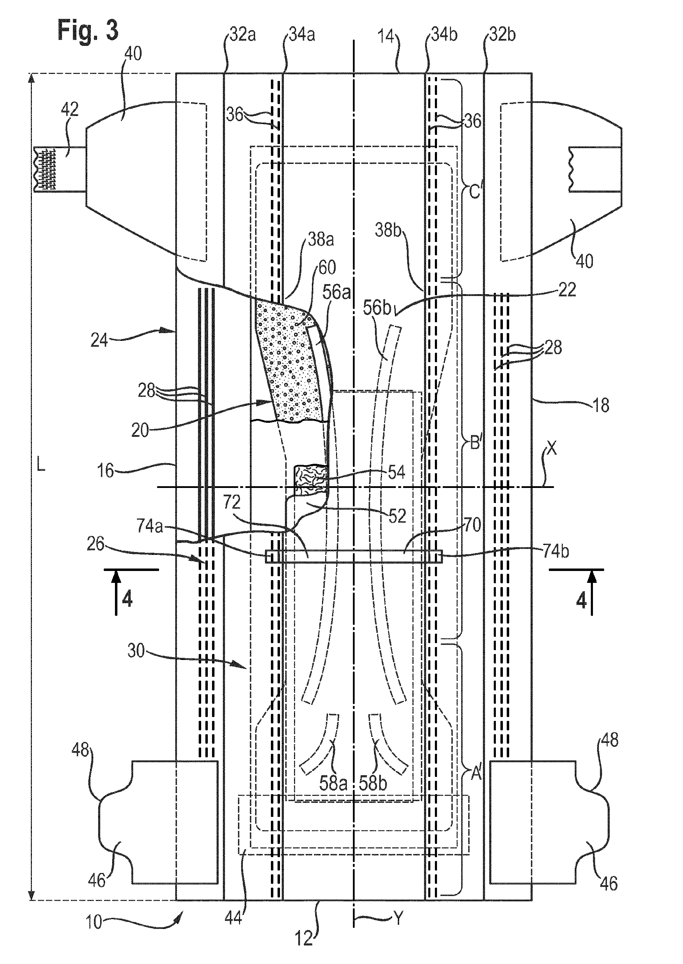

Figure 3 provides a top plane view onto the barrier facing side of a diaper 10 according to the present invention. In the central or crotch region B of the diaper and thereby the central or crotch region B' of the barrier leg cuffs, a cuff connector 70 is arranged. The cuff connector 70 is provided in form of a strip 72. This strip 72 is attached to each of the barrier leg cuffs 30a and 30b. The attachment is achieved in attachment areas 74a and 74b. The cuff connector 70 limits the freedom of movement in particular of the free flaps 38a and 38b.

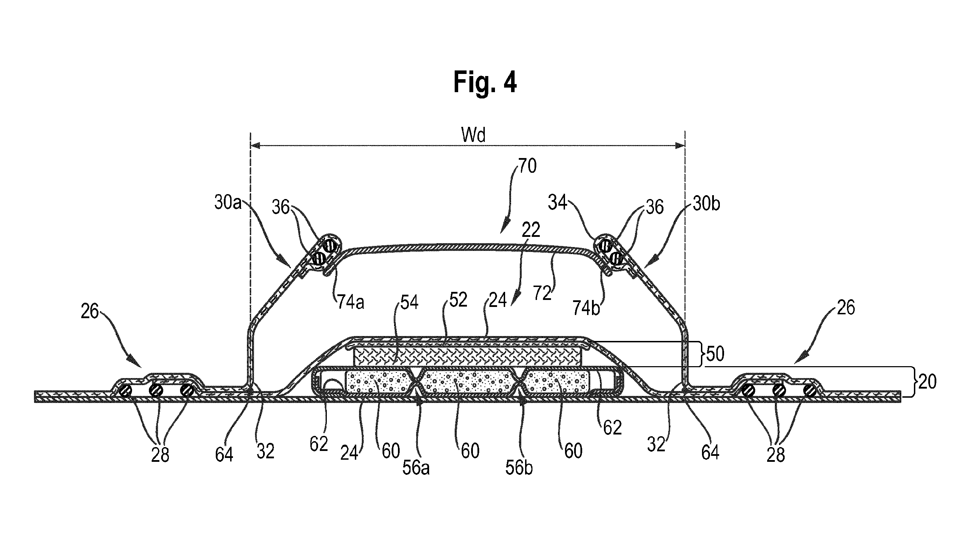

Figure 4 provides a cross sectional view taken a longline 4-4 of Figure 3. In this cross sectional view it can readibly be seen how cuff connector 70 connects distal edge 34a of barrier leg cuff 30a with distal edge 34b of barrier leg cuff 30b. Cuff connector 70 is provided in the form of a strip 72. The attachment areas 74a and 74b are provided on the inner side of barrier leg cuff 30a and 30b, this is the side of the barrier leg cuffs generally facing the topsheet 22. The respective attachment area 74a and 74b are provided adjacent to the elastics 36. They could, however, also be provided in other areas of the barrier leg cuffs 30.

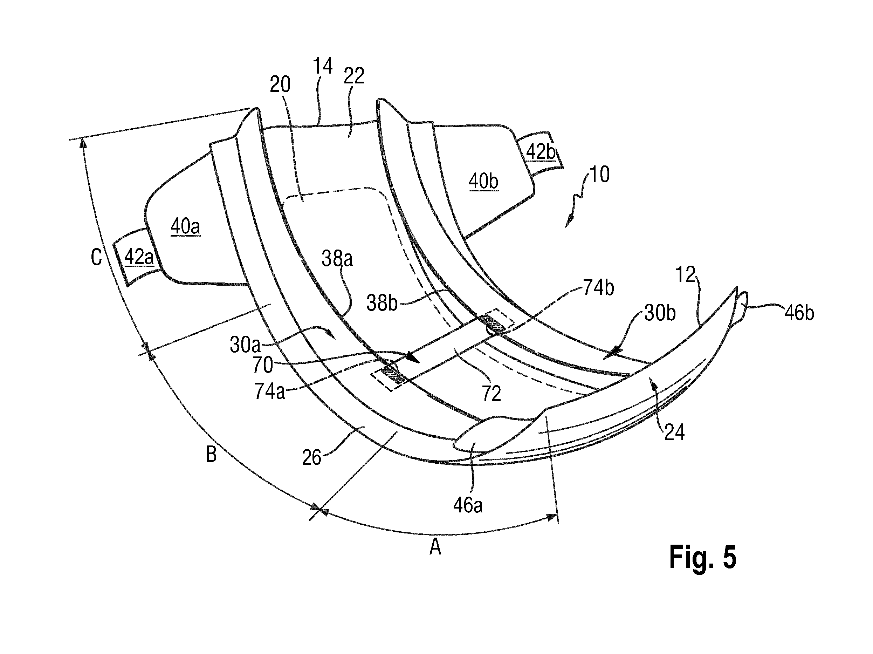

Figure 5 provides a perspective view of the diaper 10 according to the present invention. The diaper 10 is shown in a non-flat configuration. This configuration at least in part approximates the configuration which the diaper assumes when being worn. It is visible in this configuration that the barrier leg cuffs 30a and 30b stand up above the topsheet 22. Thereby, the free flaps 38a and 38b have a high freedom of movement. To a certain extend, this freedom of movement is limited by the elastics 36 (as shown in Fig. 3 and 4). However, the cuff connector 70 provides in an efficient way a further limitation of the freedom of movement. The cuff connector 70 directly connects the opposing barrier leg cuffs 30. The construction of the barrier leg cuffs 30, for example the use of elastics 36, ensures that the cuff connector 70 can achieve the described benefits without attachment to other portions of the diaper 10. The cuff connector 70 assumes a position which is defined by an equilibrium of several forces, which can be carefully designed and influenced. These forces include those exerted by the barrier leg cuffs 30a and 30b, by the elastics 36 of these cuffs and by the cuff connector itself.

Hence, the cuff connector 70 assumes this position reliably without attachment to other portions of the diaper. In particular the tension induced in the barrier leg cuffs 30 ensures that the cuff connector 70 remains in some sort of "free floating" position above the topsheet 22.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

Every document cited herein, including any cross referenced or related patent or application, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. An infant or adult diaper (10) having a longitudinal centerline (Y) and a lateral centerline (X) perpendicular to the longitudinal centerline (Y), wherein the diaper (10) comprises a topsheet (22) and a backsheet (24) and positioned therein between an absorbent core (20), and a first longitudinally extending elastic cuff (30a) and second longitudinally extending elastic cuff (30b), the first and the second elastic cuff (30a, 30b) being positioned on either longitudinal side of the topsheet (22) and opposing each other, and wherein the diaper (10) has a crotch region (B), positioned in between a front region (A) and a back region (C) and the first and the second longitudinally extending elastic cuff (30a, 30b) each have a crotch region (B'), positioned in between a front region (A') and a back region (C'), these regions of the longitudinally extending elastic cuffs (30a, 30b) being generally positioned in or adjacent to the respective regions of the diaper (10), and the first longitudinally extending elastic cuff (30a) and the second longitudinally extending elastic cuff (30b) each have a proximal edge (32), at which the respective longitudinally extending elastic cuff is attached to the diaper and the the first longitudinally extending elastic cuff (30a) and the second longitudinally extending elastic cuff (30b) each have a distal edge (34), at least the crotch region (B') of which is not attached to the diaper (10) and forms a free flap (38a, 38b) and wherein the diaper (10) comprises a cuff connector (70) being attached to the first longitudinally extending elastic cuff (30a) and the second longitudinally extending elastic cuff (30b), extending in a generally transverse direction and being able to extert a force between the first longitudinally extending elastic cuff (30a) and the second longitudinally extending elastic cuff (30b), wherein the cuff connector (10) comprises a cuff connector material and the cuff connector material:
- has a transverse strain of about 0.8 at a first cycle extension force of about 1.0 N or less, as measured according to the in-use two-cycle hysteresis test; and
- has about 30% set or less after having been extended to a transverse strain of about 0.8, as measured according to the in-use two-cycle hysteresis test; and
- is elastically extensible in the transverse direction to a strain of at least about 0.8, as measured according to the in-process two-cycle hysteresis test at 22°C

2. The diaper (10) according to claim 1, wherein the cuff connector (70) is attached at the crotch region (B') of the first longitudinally extending elastic cuff (30a) and is attached at the crotch region (B') of the second longitudinally extending elastic cuff (30b).

3. The diaper (10) according to any one of the preceeding claims, wherein the cuff connector (70) connects the first longitudinally extending elastic cuff (30a) and the second longitudinally extending elastic cuff (30b) directly.

4. The diaper (10) according to any one of the preceeding claims, wherein the cuff connector (70) is not attached to portions of the diaper (10) other than the first longitudinally extending elastic cuff (30a) and the second longitudinally extending elastic cuff (30b).

5. The diaper (10) according to any one of the preceeding claims, wherein the cuff connector (70) does not impart a substantial force onto the the first longitudinally extending elastic cuff (30a) and the second longitudinally extending elastic cuff (30b) when the diaper is in its storage configuration.

6. The diaper (10) according to any one of the preceeding claims, wherein the cuff connector (70) extends in a direction which is essentially vertical to the direction of extension of the longitudinally extending elastic cuff (30a, 30b) adjacent to the attachment area of the cuff connector (70).

7. The diaper (10) according to any one of the preceeding claims, wherein the cuff connector (70) is attached to at least one of the longitudinally extending elastic cuffs by means of an adhesive.

8. The diaper (10) according to any one of the preceeding claims, wherein the cuff connector (70) is provided in the form of a strip.

9. The diaper (10) according to claim 6, wherein the strip has a width, as measured in the longitudinal direction of the diaper (10), from about 2 mm to about 25 mm.

10. The diaper (10) according to any one of the preceeding claims, wherein the cuff connector (70) has a length, as measured in the transversal direction, from about 3 cm to about 15 cm.

11. The diaper (10) according to any one of the preceeding claims, wherein the cuff connector (70) comprises a non-woven material or is made from a non-woven material.

12. The diaper (10) according to any one of the preceeding claims, wherein the cuff connector (70) comprises an elastic material or is made from an elastic material.

13. The diaper (10) according to any one of the preceeding claims, wherein the cuff connector (70) is made of or comprises a laminate, which comprises an elastic material or a non-woven material.
